# EUROPÄISCHE PATENTSCHRIFT

(11) **EP 3 556 333 B1**
(45) Veröffentlichungstag und Bekanntmachung des Hinweises auf die Patenterteilung: **06.09.2023**
(21) Anmeldenummer: 19163026.8
(22) Anmeldetag: 15.03.2019
(51) Int. Cl.: A61F 13/20

(54) **TAMPON**
TAMPON
TAMPON

(30) Priorität: 29.03.2018 DE 102018107634
(43) Veröffentlichungstag der Anmeldung: 23.10.2019
(73) Patentinhaber: Rauscher Consumer Products GmbH, 1141 Wien (AT)
(72) Erfinder: PÖTSCH, Ernst, 2525 Schönau an der Triesting (AT); STEINLECHNER, Erik, 2500 Baden (AT)
(74) Vertreter: Seranski, Klaus

(56) Entgegenhaltungen:
- EP-A1- 1 988 202
- EP-A1- 2 417 954
- EP-A2- 2 236 110
- WO-A1-2013/093675
- DE-T2- 69 811 114

## Beschreibung

Die Erfindung betrifft einen Tampon nach dem Oberbegriff des Patentanspruchs 1.

Tampons werden schon seit langer Zeit zur Aufnahme von Körperflüssigkeiten in der Wundversorgung, maßgeblich aber im Zusammenhang mit der weiblichen Menstruation eingesetzt. Ein einfacher Tampon, bestehend aus eine Pfropfen aus einem aufsaugfähigen Material, einer Umhüllung aus Gaze, einem Rückholfaden und einem Trichter aus durchlässigem Material ist bereits in der 1936 vom Reichspatentamt herausgegebenen Patentschrift 624395 beschrieben. Die Entwicklung entsprechender Tampons ist bis heute nicht abgeschlossen. Beispielhaft wird für die einzelnen Entwicklungsschritte Bezug genommen auf die deutsche Patentschrift 804835 aus dem Jahr 1949, die deutsche Offenlegungsschrift 2722802 A1 aus dem Jahr 1977, die deutsche Patentanmeldung 3519515 A1 aus dem Jahr 1985, die internationale Patentanmeldung mit der Veröffentlichungsnummer WO 99/027878 aus dem Jahr 2000, die internationale Patentanmeldung mit der Veröffentlichungsnummer WO 01/24729 A2 aus dem Jahr 2001, die europäische Patentanmeldung mit der Veröffentlichungsnummer 2298259 A aus dem Jahr 2010 und die danach veröffentlichte europäische Patentanmeldung 2431014 A1 sowie die deutsche Patentanmeldung 10 2012 023 812 A1 aus dem Jahr 2012 und die europäische Patentanmeldung EP 3067025 A1 aus dem Jahr 2016.

Bei allen bekannten Tampons dient der Saugkörper zum Aufsaugen der Menstruationsflüssigkeit, während mit dem Hüllmaterial die strukturelle Integrität des durch Aufnahme von Menstruationsflüssigkeit aufgequollenen Saugkörpers gewährleistet wird.

Gemäß der EP 1035819 B1 werden Hüllmaterialien mit einem besonders geringen Haftreibungskoeffizienten eingesetzt. Dadurch soll das Einführen des Tampons einfacher und angenehmer gestaltet werden. Gemäß dem in der genannten Schrift beschriebenen Verfahren wird das Hüllmaterial vor der Herstellung des Tampons durch Kalandrieren geglättet, in Abschnitte geeigneter Länge geschnitten und auf ein zu einem Saugkörper aufwickelbares Materialband aufgesiegelt. Wenngleich das Einführen und Entfernen des aus der genannten Schrift bekannten Tampons vereinfacht werden kann, hat es sich gezeigt, dass die Saugfähigkeit des Saugkörpers mit diesen bekannten Tampons nicht immer vollständig ausgenutzt werden kann.

Gemäß der EP 2298259 A1 soll die Ausnutzung der Saugfähigkeit des Tampons dadurch verbessert werden, dass im Scheitelbereich der das Einführende des Tampons bildenden Kuppe eine Vertiefung gebildet wird, durch welche die dort auftreffende Körperflüssigkeit direkt in den Saugkern des Saugkörpers gelangt, so dass eine frühzeitige Sättigung des äußeren Bereichs des Saugkörpers im Kuppenbereich zuverlässig verhindert werden kann.

Gemäß der DE 10 2012 023 812 A1 kann das Ausfusseln von Fasern aus dem Saugkörper derartiger Tampons verhindert werden, indem auch die mit der Vertiefung ausgestatteten Kuppe von dem Hüllmaterial abgedeckt wird. Gemäß der EP 3067025 A1 wurde erkannt, dass es beim Verpressen des Tampons zum Erhalt einer mit einer Vertiefung versehenen und vom Hüllmaterial abgedeckten Kuppe zu einer Materialansammlung des Hüllmaterials in der Vertiefung kommen kann, welche die Ausnutzung der Saugfähigkeit des Saugkörpers wiederum beeinträchtigt.

Zur Lösung dieses Problems wird in der EP 3067025 A1 eine Weiterbildung des Tampons, dessen Einführende von einem Hüllmaterial abgedeckt ist, vorgeschlagen, die im Wesentlichen dadurch gekennzeichnet ist, dass die Kuppe einen konvexen Scheitelbereich, also einen Scheitelbereich ohne Vertiefung aufweist. Bei sämtlichen mit einem Hüllmaterial, wie es aus der EP 1035819 B1 bekannt ist, ausgestatteten Tampons muss allerdings festgestellt werden, dass die Saugfähigkeit des Saugkörpers in vielen Fällen nur unzureichend ausgenutzt wird.

Angesichts dieser Probleme im Stand der Technik liegt der Erfindung die Aufgabe zugrunde, einen Tampon bereitzustellen, mit dem die Saugfähigkeit des Saugkörpers vollständig ausgenutzt werden kann.

Erfindungsgemäß wird diese Aufgabe durch die im kennzeichnenden Teil des Patentanspruchs 1 angegebene Weiterbildung der bekannten Tampons gelöst.

Vorteilhafte Weiterbildungen der Erfindung sind in den abhängigen Patentansprüchen angegeben.

Im Rahmen der Erfindung wurde erkannt, dass die geringfügige Beeinträchtigung des Komforts beim Einführen und Entfernen des Tampons durch Erhöhung des Haftreibungskoeffizienten des Hüllvlieses im Hinblick auf den Komfortgewinn, der sich durch die erfindungsgemäß ermöglichte längere Tragezeit ergibt, ohne weiteres in Kauf genommen werden kann. Der Komfortverlust beim Einführen und Entfernen des Tampons kann ohne Beeinträchtigung des Komfortgewinns durch die längere Tragezeit in Grenzen gehalten werden, wenn der Haftreibungskoeffizient des Hüllvlieses zumindest im Bereich der Außenseite 0,7 oder weniger, vorzugsweise 0,65 oder weniger, insbesondere 0,5 oder weniger beträgt. Bei allen Ausführungsformen der Erfindung wird der Haftreibungskoeffizient entsprechend ASTM D 4918-1997 bestimmt.

Die Erfindung kann mit besonderem Vorteil bei solchen Tampons eingesetzt werden, bei denen das Hüllmaterial die Kuppe des Saugkörpers zumindest teilweise, vorzugsweise vollständig abdeckt. Bei derartigen Tampons wird das Hüllmaterial beim Verpressen verdichtet. Wenn das Hüllmaterial durch die Glättung bereits vorverdichtet ist, wird die Flüssigkeitsdurchlässigkeit so stark reduziert, dass im Bereich der Kuppe auf den Tampon auftreffende Menstruationsflüssigkeit dort kaum noch eindringen kann. Wenn aber die Glättung des Hüllmaterials im Rahmen der Erfindung nur so erfolgt, dass sich ein Haftreibungskoeffizient von 0,2 oder mehr, vorzugsweise mehr als 0,35, insbesondere mehr als 0,41 einstellt, reicht die Durchlässigkeit des Hüllmaterials auch im Bereich der Kuppe noch aus, um eine vollständige Ausnutzung der Saugfähigkeit des Saugkörpers zu ermöglichen.

Der Erhalt der Flüssigkeitsdurchlässigkeit des Hüllmaterials im Rahmen der Erfindung ermöglicht auch eine vollständige Ausnutzung der Saugfähigkeit des Saugkörpers, wenn der Tampon entsprechend der Lehre der EP 2298259 A bzw. DE 10 2012 023 812 A1 eine sich in Richtung auf das der Kuppe entgegengesetzte axiale Ende des Saugkörpers erstreckende Vertiefung in ihrem Scheitelbereich aufweist. Der Offenbarungsgehalt dieser Schriften bezüglich der Herstellung von Tampons mit einer Vertiefung im Bereich der Kuppe wird hiermit durch ausdrückliche Bezugnahme in diese Beschreibung aufgenommen.

Die strukturelle Integrität des Hüllmaterials kann auch bei einer nur mäßigen Verfestigung im Rahmen der Kalandrierung erhalten werden, wenn das Hüllmaterial Zwei-Komponenten-Fasern mit einem Faserkern und einem Fasermantel aufweist, wobei das Material des Fasermantels eine niedrigere Schmelztemperatur besitzt als das Material des Faserkerns.

Das optische Erscheinungsbild erfindungsgemäßer Tampons kann verbessert werden, wenn das Hüllmaterial Polypropylenfasern aufweist, wobei der Anteil der Polypropylenfasern bezogen auf das Gesamtgewicht des Hüllmaterials mehr als 0,5 Gew.-%, vorzugsweise 5 Gew.-% oder mehr, insbesonder 10 Gew.-% oder mehr und/oder 30 Gew.-% oder weniger, insbesondere 20 Gew.-% oder weniger beträgt. Bei der Verfestigung des Faservlieses kann es zu einer glasartigen Verschmelzung der Polypropylenfasern kommen. So kann der Glanz des Vlieses und damit auch der Glanz des Tampons, dessen äußere Begrenzungsfläche durch das Polypropylenfasern enthaltenden Vlies gebildet wird, wesentlich erhöht werden.

Wenn das Hüllmaterial den Tampon auch im Bereich der Kuppe abdeckt, kann das vollständige Aufquellen des Saugkörpers begünstigt werden, indem das Hüllmaterial im Bereich der Kuppe mit einer Schwächungslinie, wie etwa eine Perforationslinie, versehen wird, längs der das Hüllmaterial bei Aufquellen des Saugkörpers reißen kann. Diese im Rahmen der Erfindung vorgesehene Weiterbildung geht auf die Erkenntnis zurück, dass die Verdichtung bzw. Faltung des Hüllmaterials bei der Herstellung eines Tampons durch Verpressen eines Tamponrohlings ein Entfalten bzw. Aufquellen des Saugkörpers im Bereich der Kuppe behindern kann, was wiederum die Ausnutzung der Saugfähigkeit beeinträchtigt. Wenn im Bereich der Kuppe eine Schwächungslinie im Hüllmaterial vorgesehen ist, kann das Hüllmaterial im Verlauf des Aufquellens des Tampons im Bereich der Kuppe aufreißen und so ein vollständiges Aufquellen des Tampons begünstigen. Dieser Gesichtspunkt wird unabhängig von der Einstellung des Haftreibungskoeffizienten des Hüllmaterials beansprucht.

Die gerade beschriebene Schwächungslinie des Hüllmaterials kann zumindest abschnittweise parallel zur Längsachse und/oder einen spitzen Winkel mit der Längsachse des Tampons einschließend ausgeführt sein. Dabei kann sich die Schwächungslinie ausgehend von einem sich etwa senkrecht zur Längsachse des Tampons erstreckenden Rand des Hüllmaterials in Richtung auf das der Kuppe entgegengesetzte axiale Ende des Tampons, vorzugsweise über maximal 50 % der axialen Länge des Tampons, erstrecken.

Eine erfindungsgemäße Vorrichtung zum Herstellen eines Tampons ist im Wesentlichen gekennzeichnet durch eine Einrichtung zum Herstellen mindestens einer Schwächungslinie in einem die Kuppe des Tampons abdeckenden Bereich des Hüllmaterials.

Nachstehend wir die Erfindung unter Bezugnahme auf die Zeichnung, auf die hinsichtlich aller erfindungswesentlichen und in der Beschreibung nicht näher herausgestellten Einzelheiten ausdrücklich Bezug genommen wird, erläutert. In der Zeichnung zeigt:
- Fig. 1: einen Tampon gemäß einer ersten Ausführungsform,
- Fig. 2: einen Tampon gemäß einer zweiten Ausführungsform,
- Fig. 3: einen Tampon gemäß einer dritten Ausführungsform,
- Fig. 4: eine schematische Darstellung einer Vorrichtung zum Herstellen von Tampons,
- Fig. 5: einen mit der Vorrichtung gemäß Fig. 4 erhaltenes Zwischenprodukt,
- Fig. 6: eine schematische Darstellung einer Vorrichtung zum Herstellen gemäß einer zweiten Ausführungsform,
- Fig. 7: ein mit der Vorrichtung gemäß Fig. 6 erhaltenes Zwischenprodukt,
- Fig. 8: eine schematische Darstellung einer Vorrichtung zum Herstellen Tampons gemäß einer dritten Ausführungsform, und
- Fig. 9: eine Draufsicht einer Kuppe eines mit einer Vorrichtung gemäß Fig. 8 hergestellten Tampons.

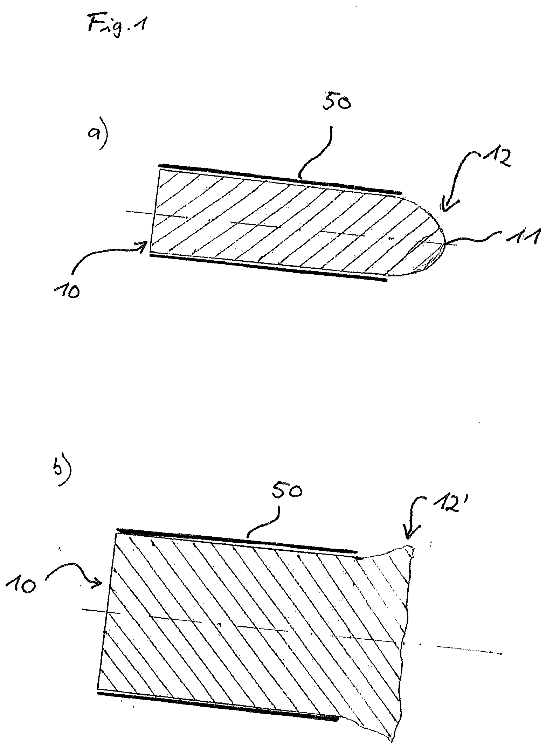

Der in Fig. 1 dargestellte Tampon umfasst einen insgesamt mit 10 bezeichneten Saugkörper, der aus einem gewickelten Watteband bestehen kann. Der Saugkörper weist an einem seiner axialen Enden eine sich in Richtung auf dieses axiale Ende 11 verjüngende Kuppe 12 auf. Der Saugkörper 10 ist bei der in Fig. 1 dargestellten Ausführungsform der Erfindung bis zu dem Bereich der Kuppe 12 von einem Hüllmaterial 50 abgedeckt. Die Kuppe selbst ist nicht von dem Hüllmaterial abgedeckt. Wenn der Saugkörper während des Gebrauchs Menstruationsflüssigkeit aufsaugt, quillt er auf und nimmt die in Fig. 1b schematisch angegebene Form an. Dabei wird die strukturelle Integrität des Saugkörpers außerhalb der Kuppe 12 durch das Abdeckmaterial 50 gewährleistet. Ein behinderungsfreies Ausdehnen des Saugkörpers in dem Bereich, in dem dieser von dem Abdeckmaterial abgedeckt ist, wird üblicherweise dadurch begünstigt, dass das Hüllmaterial 50 bei der Herstellung des Tampons in in axialer Richtung verlaufende Rillen (nicht dargestellt) gepresst wird.

Im Bereich der Kuppe 12 wird das Aufquellen des Saugkörpers nicht durch das Hüllmaterial beeinflusst. Der Saugkörper kann dort weiter aufquellen. Dadurch wird die vollständige Ausnutzung der Saugfähigkeit des Saugkörpers 10 begünstigt. Allerdings besteht so das Risiko, dass sich Fasern aus dem Saugkörper 10 lösen und nach dem Gebrauch in der Körperhöhle verbleiben.

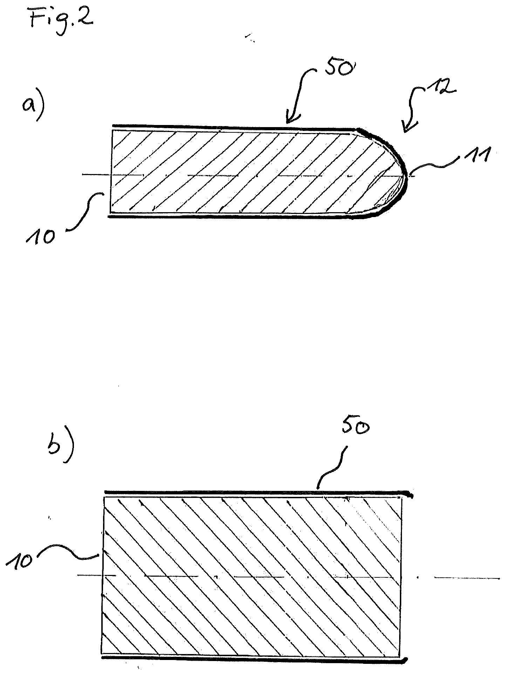

Dieser Mangel kann durch einen Tampon, wie er in Fig. 2 dargestellt ist, überwunden werden. Bei diesem Tampon erstreckt sich das Hüllmaterial über die gesamte Länge des Saugkörpers 10 und deckt auch die Kuppe 12 bis zu deren Spitze 11 ab. Während des Gebrauchs dehnt sich der Saugkörper 10 über seine gesamte Länge gleichmäßig aus und wird insgesamt vom Hüllmaterial 50 zusammengehalten. Allerdings wird das weitere Aufquellen des Saugkörpers 10 auch im Bereich der Kuppe 12 durch das Hüllmaterial 50 begrenzt. Das kann dazu führen, dass die Saugfähigkeit des Saugkörpers nicht vollständig ausgenutzt wird.

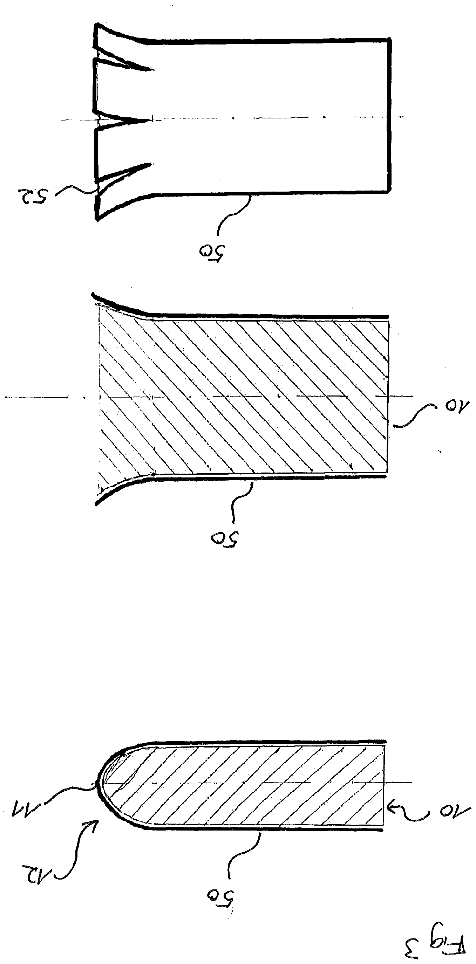

Dieser Mangel wird durch einen Tampon gemäß Fig. 3 beseitigt. Wie in Fig. 3c erkennbar ist, weist das Hüllmaterial 50 im Bereich der Kuppe 12 sich ausgehend von einem Rand des Hüllmaterials in Richtung auf das entgegengesetzte Ende des Tampons erstreckende Schwächungslinien 52 auf, längs denen das Hüllmaterial während des Gebrauchs aufreißen kann. Die Schwächungslinien erstrecken sich über etwa 20 % der gesamten axialen Länge des Saugkörpers 10. Die Schwächungslinien 52 sind in den Schnittdarstellungen gemäß Fig. 3a und 3b nicht erkennbar. In Fig. 3b ist lediglich erkennbar, dass die Schwächungslinien das Aufquellen des Saugkörpers im Bereich der Kuppe 12 soweit ermöglichen, dass der Saugkörper im Bereich der Kuppe 12 weiter aufquillt als in dem verbleibenden Bereich. Mit dem in Fig. 3 dargestellten Tampon kann sowohl die Saugfähigkeit des Saugkörpers nahezu vollständig ausgenutzt werden als auch das Lösen von Fasern während des Gebrauchs begrenzt werden.

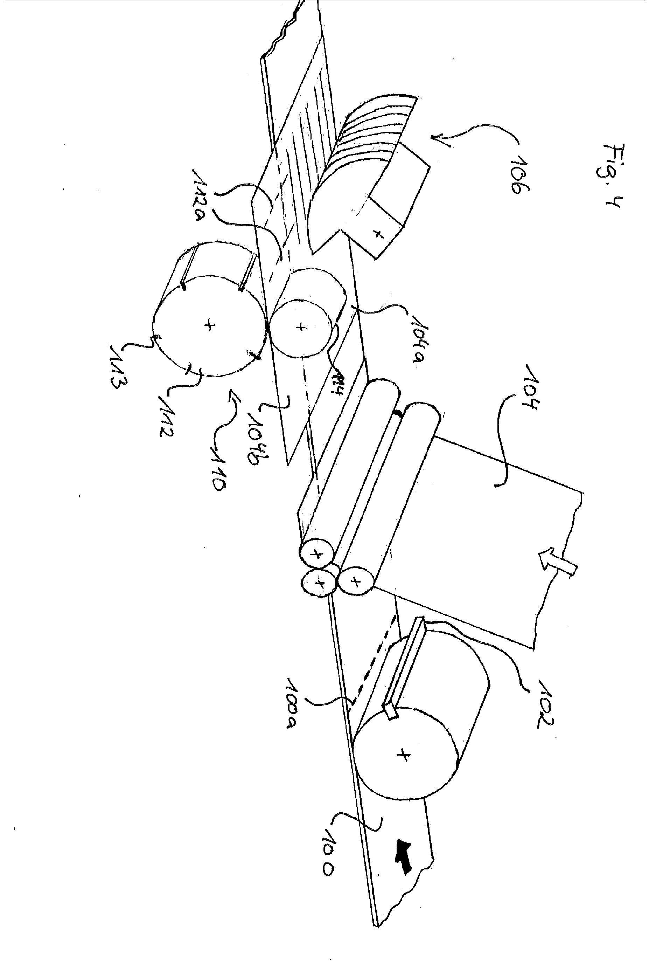

Gemäß Fig. 4 wird bei der Herstellung eines Tampons gemäß Fig. 3 zunächst ein Watteband 100 in eine Tamponmaschine eingezogen und mit einem Wattemesser 102 auf die zur Herstellung einzelner Tampons benötigte Länge geschnitten. Die Breite des Wattebands 100 beträgt dabei etwa 40 bis 65 mm. Die einzelnen Wattebandabschnitte, welche zur Herstellung eines Tampons benötigt werden, sind in Fig. 4 anhand der gestrichelten Linien 100a angedeutet.

Nach dem Schneiden des Wattebands 100 zu Wattebandabschnitten mit der benötigten Länge wird ein Hüllvlies 104 mit etwa der halben Länge der Wattebandabschnitte derart versetzt um etwa 50 bis 80 % der Länge eines Wattebandabschnitts unter Druck und Hitze mit Hilfe einer Siegelbacke 106 auf das Watteband 100 aufgesiegelt, dass sich ein Querüberstand 104a über dem Wattebandabschnitt ergibt, auf den das Hüllvlies mit Hilfe der Siegelbacke 106 aufgesiegelt ist.

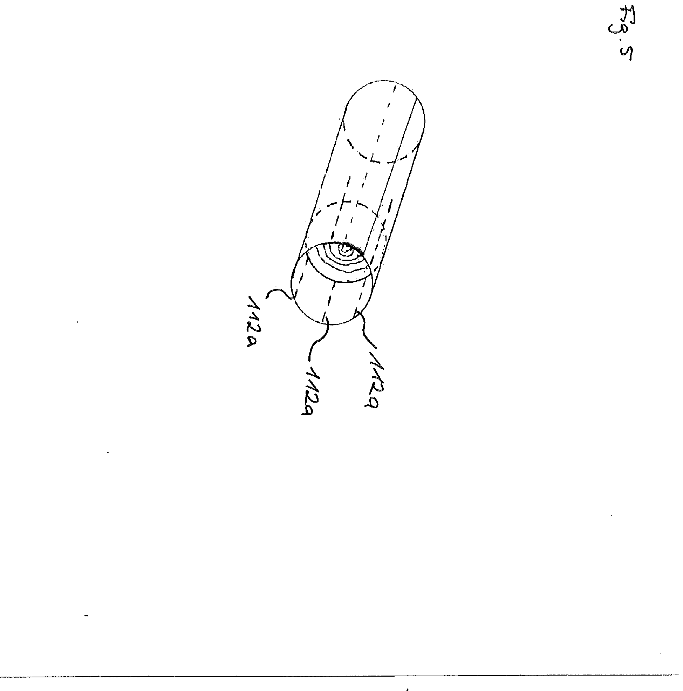

Beim Aufwickeln des Wattebands 100 zu einem Vorprodukt gemäß Fig. 5 kommt der Querüberstand 104a auf einem auf dem Watteband 100 aufgesiegelten Bereich des Hüllvlieses 104 zu liegen und kann darauf befestigt werden. Die Breite des Hüllvlieses 104 übersteigt die Breite des Wattebands 100 um 13 bis 63 %. Bei bündiger Anlage des Hüllvlieses 104 an einem Längsrand des Wattebands 100 ergibt sich im Bereich des anderen Längsrands des Wattebands 100 ein Längsüberstand 104b von 13 bis 63 %, insbesondere 20 bis 50 %, der bei der anhand der Zeichnung erläuterten Ausführungsform der Erfindung etwa 8 bis 20 mm beträgt.

Im Bereich des Längsüberstands 104b werden bei der Vorrichtung gemäß Fig. 4 mit Hilfe einer Perforationswalze 112 Schwächungslinien gebildet, wie bei 112a angedeutet. Die Perforationswalze 112 ist bezüglich einer parallel zu einer Drehachse des Wattemessers 102 verlaufenden Drehachse drehbar. Auf der Umfangsfläche der Perforationswalze 112 sind Perforationsmesser 113 in in Umfangsrichtung gleichbleibenden Abständen angeordnet. Mit den Perforationsmessern 113 werden die Perforationslinien 112a im Längsüberstand 104b gebildet. Dazu wird der Längsüberstand 104b bei der Herstellung erfindungsgemäßer Produkte nach Aufsiegeln des Hüllmaterials 104 auf das Watteband 100 zwischen der Perforationswalze 112 und einer Gegenwalze 114 geführt. Nach Aufwickeln des Wattebands 100 mit dem darauf angebrachten und mit Perforationslinien 112a versehenen Hüllvlies ergibt sich ein Zwischenprodukt gemäß Fig. 5. Der Längsüberstand 104b weist Perforationslinien 112a auf, welche im fertiggestellten, d. h. verpressten Tampon den Perforationslinien 52 gemäß Fig. 3 entsprechen.

Hinsichtlich weiteren Einzelheiten von Vorrichtung zum Herstellen Tampons und entsprechender Verfahren wird Bezug genommen auf den Offenbarungsgehalt der DE 10 2012 023 812 A1, der hiermit durch ausdrückliche Inbezugnahme in diese Beschreibung aufgenommen wird.

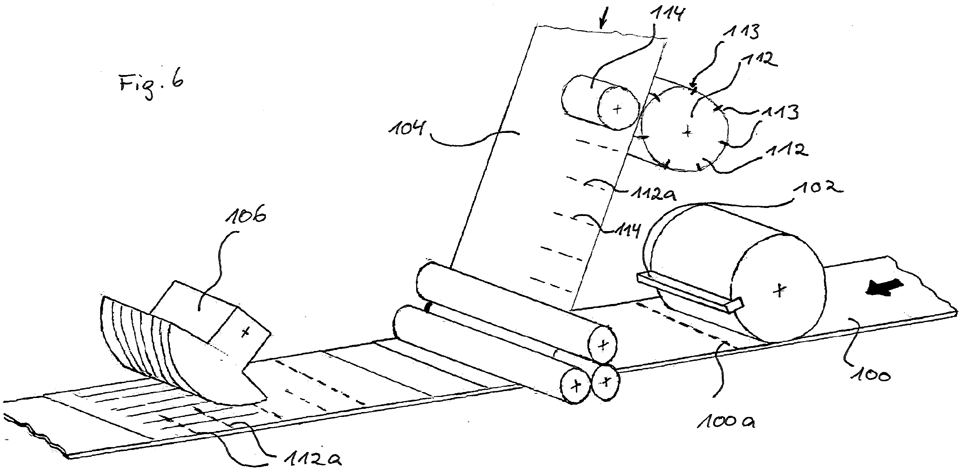

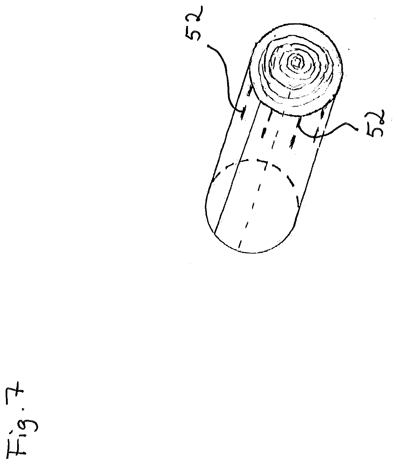

Bei der Ausführungsform gemäß Fig. 6 wird das Hüllvlies 104 ohne Längsüberstand auf das Watteband 100 aufgesiegelt. Die Perforationslinien werden mit Hilfe der Perforationswalze 112 vor Anlegen des Hüllvlieses 104 an das Watteband 100 hergestellt. Mit den in Fig. 6 dargestellten Vorrichtungen können Vorprodukte gemäß Fig. 7 hergestellt werden, bei denen die Perforationslinien 52 im Bereich des den Saugkörper abdeckenden Endes des Hüllmaterials angeordnet sind.

Bei den in der Zeichnung dargestellten Ausführungsformen der Erfindung erstrecken sich die Schwächungslinien im Bereich des Hüllmaterials etwa parallel zur Tamponachse. Es ist allerdings auch an solche Ausführungsformen gedacht, bei denen die Schwächungslinien die Tamponachse etwa wendelförmig umlaufen. Solche Perforationslinien können erhalten werden, wenn die Perforationsmesser 113 der Perforationswalze 112 entsprechend umlaufend ausgeführt sind.

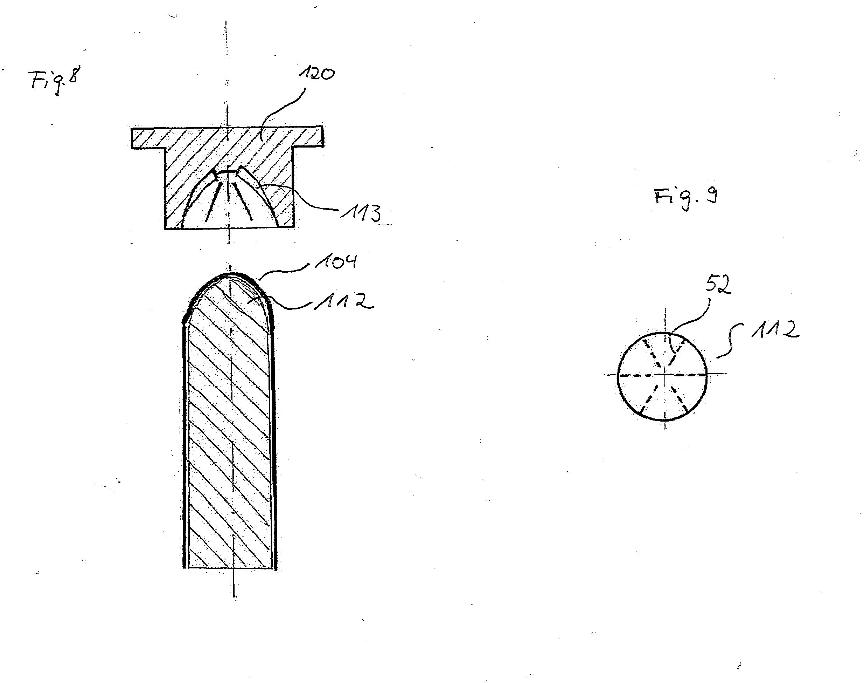

Mit einer schematisch in Fig. 8 dargestellten Vorrichtung werden die als Perforationslinien ausgeführten Schwächungslinien im Bereich der Tamponkuppe mit einem sogenannten Kuppenformer 120 erhalten. Mit Hilfe eines solchen Kuppenformers wird ein bereits radial verpresster Tamponrohling mit der gewünschten Kuppenform versehen. Dazu wird der Kuppenformer in axialer Richtung auf ein axiales Endes des Tamponrohlings aufgesetzt.

Der in Fig. 8 dargestellte Kuppenformer 120 ist auf seiner der Kuppenformung dienenden inneren Begrenzungsfläche mit Perforationsmessern 113 ausgestattet, welche im Bereich der Kuppe 12 Perforationslinien in dem Hüllmaterial 104 erzeugen. Die so erhaltenen Perforationslinien sind in der Draufsicht auf einen erfindungsgemäßen Tampon bei 52 angedeutet. Bei dieser Ausführungsform der Erfindung sind die Perforationslinien so ausgeführt, dass sie nicht unmittelbar von einem Rand des Hüllmaterials ausgehen. Sie erstrecken sich aber in Richtung auf das der Kuppe entgegengesetzte Ende des Tampons.

### Bezugszeichenliste

- 10: Saugkörper
- 11: Spitze
- 12: Kuppe
- 50: Hüllmaterial
- 52: Schwächungslinie
- 100: Watteband
- 102: Wattemesser
- 104: Hüllvlies
- 106: Siegelbacke
- 104a: Querüberstand
- 104b: Längsüberstand
- 112: Perforationswalze
- 112a: Perforationslinien
- 113: Perforationssmesser
- 114: Gegenwalze
- 120: Kuppenformer

## Patentansprüche

1. Tampon zum Einführen in eine Körperhöhle, insbesondere in die weibliche Scheide, mit einem in axialer und/oder radialer Richtung verpressten Saugkörper, der eine sich in Richtung auf eines seiner axialen Enden verjüngende Kuppe aufweist, und einem die äußere Begrenzungsfläche des Saugkörpers zumindest teilweise abdeckenden, thermoplastische Fasern aufweisenden, flüssigkeitsdurchlässigen Hüllmaterial, das vorzugsweise ein Hüllvlies aufweist, wobei der Haftreibungskoeffizient des Hüllmaterials gemessen entsprechend ASTM D 4918-1997, zumindest auf der dem Saugkörper abgewandten Außenseite mehr als 0,2, beträgt, **dadurch gekennzeichnet, dass** die Kuppe mit einer zumindest teilweise, vorzugsweise vollständig von dem Hüllmaterial abgedeckten und sich in Richtung auf das entgegengesetzte axiale Ende des Saugkörpers erstreckenden Vertiefung in ihrem Scheitelbereich ausgestattet ist.

2. Tampon nach Anspruch 1, **dadurch gekennzeichnet, dass** der Haftreibungskoeffizient des Hüllmaterials zumindest im Bereich der Außenseite 0,7 oder weniger, vorzugsweise 0,65 oder weniger, insbesondere 0,5 oder weniger beträgt.

3. Tampon nach einem der vorhergehenden Ansprüche, **dadurch gekennzeichnet, dass** das Hüllmaterial die Kuppe des Saugkörpers zumindest teilweise, vorzugsweise vollständig abdeckt.

4. Tampon nach einem der vorhergehenden Ansprüche, **dadurch gekennzeichnet, dass** das Hüllmaterial Zwei-Komponenten-Fasern mit einem Faserkern und einem Fasermantel aufweist, wobei das Material des Fasermantels eine niedrigere Schmelztemperatur aufweist als das Material des Faserkerns.

5. Tampon nach einem der vorhergehenden Ansprüche, **dadurch gekennzeichnet, dass** das Hüllmaterial im Bereich der Kuppe eine Schwächungslinie, wie etwa eine Perforationslinie, aufweist, längs der das Hüllmaterial bei Aufquellen des Saugkörpers reißen kann.

6. Tampon nach Anspruch 5, **dadurch gekennzeichnet, dass** sich die Schwächungslinie zumindest abschnittweise etwa parallel zur Längsachse und/oder einen spitzen Winkel mit der Längsachse einschließend erstreckt.

7. Tampon nach Anspruch5 oder 6, **dadurch gekennzeichnet, dass** sich die Schwächungslinie ausgehend von einem sich etwa senkrecht zur Längsachse des Tampons erstreckenden Rands des Hüllmaterials in Richtung auf das der Kuppe entgegengesetzte axiale Ende des Tampons erstreckt.

8. Vorrichtung zum Herstellen eines Tampons nach einem der vorhergehenden Ansprüche mit einer Wickeleinrichtung zum Herstellen eines Wickels aus einer Materialbahn aus saugfähigem Material und einer Presseinrichtung zum Verpressen des Wickels sowie einer Kuppenformeinrichtung zur Bildung einer Kuppe (12) des Tampons, **gekennzeichnet durch** eine Einrichtung (112, 113, 114) zum Erzeugen einer Schwächungslinie in einem die Kuppe (12) abdeckenden Bereich des Hüllmaterials (50) vor und/oder nach und/oder während der Verpressung.

## Claims

1. A tampon for insertion into a body cavity, in particular into a woman's vagina, comprising an absorbent body compressed in an axial and/or radial direction which has a tip tapering towards one of its axial ends, and comprising a liquid-permeable cladding material which has thermoplastic fibres and at least partially covers the outer boundary of the absorbent body, said cladding material preferably comprising a cladding nonwoven material, wherein the coefficient of static friction of the cladding material as measured in accordance with ASTM D 4918-1997, at least on the outer side facing away from the absorbent body, is more than 0.2, **characterised in that** the tip is provided, in its apex region, with a recess which proceeds towards the opposite end of the absorbent body and is covered by the cladding material at least partially, preferably fully.

2. The tampon according to claim 1, **characterised in that** the coefficient of static friction of the cladding material, at least in the region of the outer side, is 0.7 or less, preferably 0.65 or less, in particular 0.5 or less.

3. The tampon according to one of the preceding claims, **characterised in that** the cladding material covers the tip of the absorbent body at least partially, preferably fully.

4. The tampon according to one of the preceding claims, **characterised in that** the cladding material has two-component fibres with a fibre core and a fibre cladding, wherein the material of the fibre cladding has a lower melting temperature than the material of the fibre core.

5. The tampon according to one of the preceding claims, **characterised in that** the cladding material has a line of weakness, such as a perforation line, in the region of the tip, along which the cladding material can tear as the absorbent body swells.

6. The tampon according to claim 5, **characterised in that** line of weakness extends at least in part approximately parallel to the longitudinal axis and/or forms an acute angle with the longitudinal axis.

7. The tampon according to claim 5 or 6, **characterised in that** the line of weakness extends from an edge of the cladding material running approximately perpendicular to the longitudinal axis of the tampon and proceeds towards the axial end of the tampon opposite the tip.

8. An apparatus for producing a tampon according to one of the preceding claims, comprising: a winding device for producing a winding from a material web made of absorbent material, a pressing device for compressing the winding, and a tip moulding device for forming a tip (12) on the tampon, **characterised by** a device (112, 113, 114) for producing a line of weakness in an area of the cladding material (50) covering the tip (12) before and/or after and/or during compressing.

## Revendications

1. Tampon destiné à être introduit dans un orifice corporel, notamment le vagin, comportant : un corps absorbant qui est comprimé dans la direction axiale et/ou radiale et présente une calotte qui va en rétrécissant en direction d'une de ses extrémités axiales ; et un matériau d'enveloppe perméable aux liquides, recouvrant au moins partiellement la surface extérieure du corps absorbant et présentant des fibres thermoplastiques, de préférence un non-tissé, le coefficient de frottement d'adhérence dudit matériau d'enveloppe mesuré selon ASTM D 4918-1997 étant de plus de 0,2, au moins sur la face extérieure détournée du corps absorbant, **caractérisé en ce que** la calotte est dotée, à son sommet, d'un creux qui s'étend en direction de l'extrémité axiale opposée du corps absorbant et qui est recouvert au moins partiellement et de préférence totalement par le matériau d'enveloppe.

2. Tampon selon la revendication 1, **caractérisé en ce que** le coefficient de frottement d'adhérence du matériau d'enveloppe est, au moins dans la zone de la face extérieure, de 0,7 ou moins, de préférence de 0,65 ou moins, notamment de 0,5 ou moins.

3. Tampon selon l'une quelconque des revendications précédentes, **caractérisé en ce que** le matériau d'enveloppe recouvre au moins partiellement et de préférence totalement la calotte du corps absorbant.

4. Tampon selon l'une quelconque des revendications précédentes, **caractérisé en ce que** le matériau d'enveloppe présente des fibres à deux composants comportant un coeur et une gaine, le matériau de la gaine présentant une température de fusion inférieure à celle du matériau du coeur.

5. Tampon selon l'une quelconque des revendications précédentes, **caractérisé en ce que** le matériau d'enveloppe présente, dans la zone de calotte, une ligne d'affaiblissement telle qu'une ligne de perforation, le long de laquelle le matériau d'enveloppe peut se déchirer lors du gonflement du corps absorbant.

6. Tampon selon la revendication 5, **caractérisé en ce que** la ligne d'affaiblissement s'étend au moins par sections de manière sensiblement parallèle à l'axe longitudinal et/ou forme un angle aigu avec l'axe longitudinal.

7. Tampon selon la revendication 5 ou 6, **caractérisé en ce que** la ligne d'affaiblissement s'étend à partir d'un bord du matériau d'enveloppe sensiblement perpendiculaire à l'axe longitudinal du tampon, en direction de l'extrémité axiale du tampon qui est opposée à la calotte.

8. Dispositif de fabrication d'un tampon selon l'une quelconque des revendications précédentes, comprenant un dispositif enrouleur pour la fabrication d'un enroulement à partir d'une bande de matière absorbante et un dispositif de compression permettant de comprimer l'enroulement, ainsi qu'un dispositif de formage de calotte permettant de former une calotte (12) du tampon, **caractérisé par** un dispositif (112, 113, 114) de production d'une ligne d'affaiblissement dans une zone du matériau d'enveloppe (50) recouvrant la calotte (12) avant et/ou après et/ou pendant la compression.
